# EUROPÄISCHE PATENTSCHRIFT

(11) **EP 0 808 619 B1**
(45) Veröffentlichungstag und Bekanntmachung des Hinweises auf die Patenterteilung: **10.09.2003**
(21) Anmeldenummer: 97107605.4
(22) Anmeldetag: 09.05.1997
(51) Int. Cl.: A61F 2/38

(54) **Ellbogengelenk-Endoprothese**
Elbow joint endroprosthesis
Endoprothèse de l'articulation du coude

(30) Priorität: 22.05.1996 DE 19620525
(43) Veröffentlichungstag der Anmeldung: 26.11.1997
(73) Patentinhaber: Waldemar Link (GmbH & Co.), 22339 Hamburg (DE)
(72) Erfinder: Engelbrecht, E. Dr. med., D-22301 Hamburg (DE); Keller, Arnold, 23863 Kayhude (DE)
(74) Vertreter: Glawe. Delfs. Moll

(56) Entgegenhaltungen:
- DE-A- 3 940 728
- DE-A- 4 331 282
- US-A- 5 133 762

## Beschreibung

Die Erfindung betrifft eine Ellbogengelenk-Endoprothese nach dem Oberbegriff des Anspruchs 1.

Aus der DE-OS 39 40 728 der Anmelderin ist bereits eine Ellbogengelenk-Endoprothese dieser Art bekannt, bei der ein Radiuskopf des Radiusteils in einem Gleitkörper verschwenkbar gelagert ist, der mit seitlichem Spiel in einer Pfanne gelagert ist, die auf der Unterseite eines am Ulnateil angeformten Auslegers oder Querstegs ausgebildet ist. Dadurch wird bewirkt, daß einerseits das Radiusteil beim Beugen und Strekken des Unterarms zusammen mit dem Ulnateil um die Schwenkachse des Schwenklagers verschwenkt wird und andererseits Relativbewegungen des Radiusteils gegenüber dem Ulnateil möglich sind, beispielsweise während eines Verdrehens des Unterarms um seine Längsachse, um zu verhindern, daß das Radiusteil dabei einer Torsions- oder Biegebelastung unterworfen wird, die zu einem Lockern seines Schaftes führen können.

Zwar hat sich diese Konstruktion grundsätzlich gut bewährt, da sie die Realisierung von Bewegungsabläufen ermöglicht, die den natürlichen Verhältnissen im Ellbogengelenk weitestgehend entsprechen. Andererseits hat sich bei Versuchen jedoch gezeigt, daß die Bewegungen des Radiusteils gegenüber dem Ulnateil weiter verbessert werden können, insbesondere im Hinblick auf eine sicherere Halterung des Radiuskopfes in bezug auf die Pfanne und eine bessere Einführung der Schwenkbewegungen, ohne einen Verzicht auf Querbeweglichkeit in der Pfanne.

Diese Aufgabe wird durch die Merkmale des Anspruchs 1 gelöst. Durch die erfindungsgemäße Bereitstellung zweier getrennter Artikulationsflächen, nämlich einer teilsphärischen Gleitfläche für Schwenkbewegungen zwischen dem Radiuskopf und dem Gleitkörper einerseits und einer im wesentlichen ebenen Gleitfläche für Querbewegungen zwischen dem Gleitkörper und der Pfanne andererseits, ergibt sich eine bessere Einführung der Schwenkbewegungen zwischen dem Radiuskopf und dem Ulnateil, ohne daß auf die Querbeweglichkeit des Radiuskopfes innerhalb der Pfanne verzichtet werden muß. Weiter kann der Gleitkörper vom Muskel- und Bänderapparat besser in der Pfanne innerhalb von deren einen Anschlag bildenden Wand festgehalten werden, so daß die Wahrscheinlichkeit einer Luxation im Bereich der Lagerung des Radiuskopfes bei extremen Beanspruchungen sinkt. Durch die Vergrößerung der Artikulationsflächen infolge der erfindungsgemäßen Anordnung werden zudem die Flächenpressungen zwischen den aufeinander gleitenden Oberflächen und damit der Verschleiß der aus dem weicheren Kunststoffmaterial bestehenden Prothesenkomponente reduziert.

Eine bevorzugte Ausgestaltung der Erfindung sieht vor, daß der Gleitkörper senkrecht zu einer Mittelachse der Pfanne mit zwei Freiheitsgraden beweglich ist, so daß er sich in der Pfanne sowohl in Richtung eines Ulnaschaftes des Ulnateils als auch quer zu dieser Richtung verschieben kann. Dies wird vorzugsweise dadurch erreicht, daß der mit seiner ebenen Oberseite gegen einen entsprechend eben ausgebildeten Boden der Pfanne anliegende Gleitkörper seitliche Abmessungen aufweist, die kleiner als die entsprechenden inneren Abmessungen der Pfanne sind, so daß zwischen einer den Boden begrenzenden umlaufenden Innenwand der Pfanne und einer seitlichen Umfangsfläche des Gleitkörpers allseitig ein Spiel von mindestens 1 mm oder vorzugsweise etwas mehr vorhanden ist, wenn der Gleitkörper in seiner Ruhelage bei unverdrehtem Unterarm mittig in die Pfanne eingreift. Der Gleitkörper und die Innenwand der Pfanne sind vorzugsweise zu ihren jeweiligen Mittelachsen rotationssymmetrisch, wobei diese bei einer mittigen Anordnung des Gleitkörpers in der Pfanne zweckmäßig miteinander fluchten. Es ist jedoch auch möglich, asymmetrische Ausbildungen insbesondere für die Innenwand der Pfanne zu wählen. So kann zweckmäßigerweise die Innenwandung auch in Form eine Ellipse ausgebildet sein. Entscheidend bei der Ausbildung der Innenwand ist die Möglichkeit, daß der Gleitkörper mit seinen Begrenzungen so an der Innenwand der Pfanne geführt wird, daß sich der Gleitkörper in der Pfanne nicht festklemmen kann. Zu diesem Zwecke ist die Innenwand der Pfanne mit stetig ineinander übergehenden Abschnitten ausgebildet, von denen jeder für eine flächige Anlage des Gleitkörpers vorgesehen ist.

Die Zuordnung des Gleitkörpers zur Pfanne ist zweckmäßigerweise abhängig von den jeweiligen anatomischen Gegebenheiten eines mit der Prothese zu versorgenden Patienten und einer jeweiligen Kipplage des Radiusteils. Von diesen Faktoren hängt auch ab, in welcher Lage sich der Gleitkörper bezüglich der Pfanne befindet, wenn er in seiner Ruhelage bei unverdrehtem Unterarm ist. Auch in dieser Ruhelage kann der Gleitkörper sich außerhalb der Pfannenmitte befinden.

Gemäß einer weiteren bevorzugten Ausgestaltung der Erfindung weist der Gleitkörper eine an eine kugelförmige Gestalt des Radiuskopfes angepaßte teilsphärische Ausnehmung auf, in welche der Radiuskopf im wesentlichen spielfrei eingreift, so daß die äußere Oberfläche des Radiuskopfes und die innere Oberfläche der Ausnehmung gegeneinander anliegen und aufeinander gleiten, wobei sie die Artikulationsfläche für die Schwenkbewegung des Radiuskopfes gegenüber dem Ulnateil bilden. Eine weitere bevorzugte Ausgestaltung der Erfindung sieht vor, daß die teilsphärische innere Oberfläche der Ausnehmung etwas größer als die Oberfläche einer Halbkugel ist. Dadurch wird der Radiuskopf im Gleitkörper festgehalten und eine axiale Bewegung zwischen dem Gleitkörper und dem Radiuskopf verhindert. Der Gleitkörper besteht zweckmäßig aus einem körperverträglichen Kunststoffmaterial vorzugsweise aus Polyethylen hoher Dichte (HDPE). Dieses Material weist eine ausreichende Elastizität auf, die es ermöglicht, den Radiuskopf in der Ausnehmung einzurasten, indem die Wand der Ausnehmung um eine Austrittsöffnung für einen an den Radiuskopf angrenzenden schlanken Hals des Radiusteils herum durch axialen Druck auf das Radiusteil elastisch aufgeweitet wird, bis der Radiuskopf in die Ausnehmung eintritt. Infolge der elastischen Rückstellkraft nimmt der verformte Wandbereich anschließend wieder seine Ausgangslage ein.

Die Pfanne ist vorzugsweise an der Unterseite eines Auslegers ausgebildet, der zu einem Ulnaschaft des Ulnateils und zur Schwenkebene des Schwenklagers im wesentlichen senkrecht ist und der zweckmäßig einstückig mit dem Ulnateil verbunden ist, so daß der Ausleger und damit die Pfanne gemeinsam mit dem Ulnateil gegenüber dem Humerusteil verschwenkbar sind. Das Ulnateil und der Ausleger bestehen vorzugsweise ebenso wie das Humerusteil und das Radiusteil mit dem Radiuskopf aus einem körperverträglichen metallischen Implantatmaterial, vorzugsweise aus CoCrMo, so daß es in Verbindung mit dem aus HDPE bestehenden Gleitkörper im Bereich der ebenen Artikulationsfläche zwischen dem Gleitkörper und dem Boden der Pfanne ebenso wie im Bereich der teilsphärischen Artikulationsfläche zwischen dem Radiuskopf und dem Gleitkörper zu einer verschleißmindernden Materialpaarung CoCrMo/HDPE kommt.

Im folgenden wird die Erfindung anhand eines in der Zeichnung dargestellten Ausführungsbeispiels näher erläutert. Es zeigen:

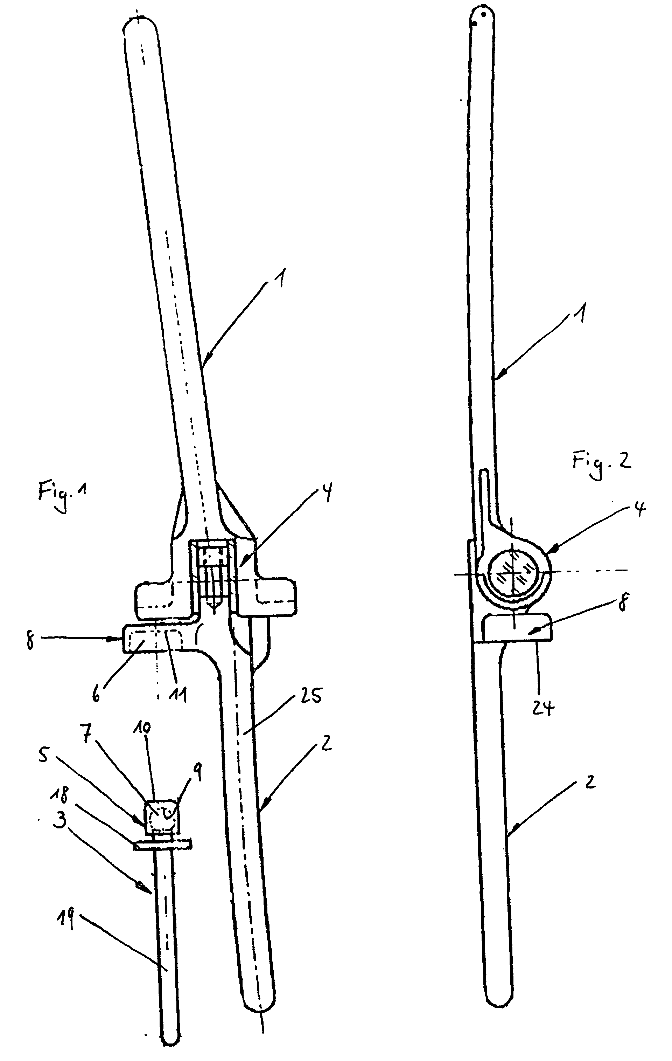

- Fig. 1:: eine teilweise auseinandergezogene Vorderseitenansicht einer erfindungsgemäßen Ellbogengelenk-Endoprothese;
- Fig. 2:: eine Seitenansicht des Humerusteils und des Ulnateils der Ellbogengelenk-Endoprothese aus Fig. 1;
- Fig. 3:: einen Längsschnitt durch den in die Pfanne des Ulnateils eingesetzten Gleitkörper und den im Gleitkörper eingerasteten Radiuskopf;
- Fig. 4:: eine teilweise geschnittene vereinfachte Vorderseitenansicht der im Knochen implantierten Ellbogengelenk-Endoprothese.

Die in der Zeichnung dargestellte Ellbogengelenk-Endoprothese besteht im wesentlichen aus einem Humerusteil 1, einem in einem Schwenklager 4 des Humerusteils 1 schwenkbar gelagerten Ulnateil 2 sowie einem Radiusteil 3, dessen kugeliger Radiuskopf 7 in eine teilsphärische Ausnehmung 9 eines Gleitkörpers 5 eingesetzt ist, der wiederum in einer Pfanne 6 auf der Unterseite eines seitlich über das Ulnateil 2 überstehenden Auslegers 8 querverschiebbar gelagert ist.

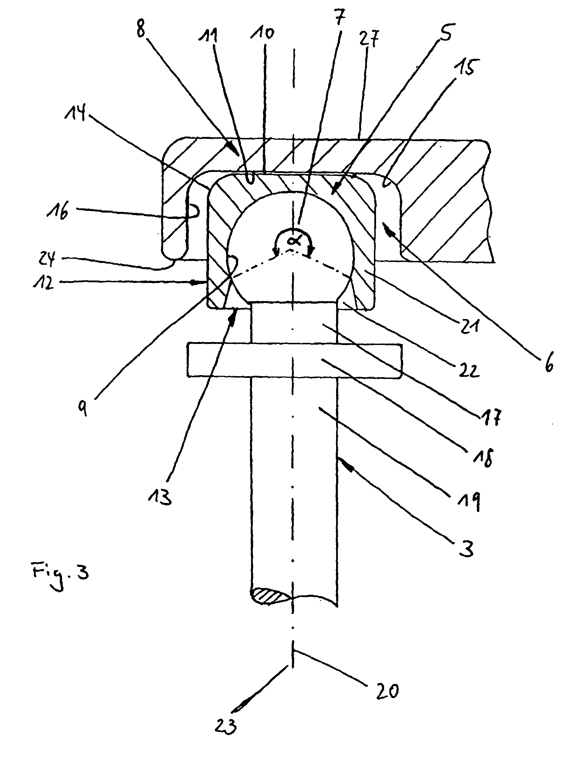

Wie am besten aus Fig. 3 ersichtlich ist, besitzt der aus Polyethylen hoher Dichte (HDPE) bestehende Gleitkörper 5 eine im wesentlichen ebene Oberseite 10, die flächig gegen einen nach unten weisenden ebenen Boden 11 der Pfanne 6 anliegt, eine zur Oberseite 10 senkrechte zylindrische Umfangsfläche 12, sowie eine dem Radiusteil 3 zugewandte Unterseite 13, zu der hin sich die zur Aufnahme des Radiuskopfes 7 dienende Ausnehmung 9 öffnet. Ein zwischen der Umfangsfläche 12 und der Oberseite 8 umlaufender Rand 14 ist abgerundet, wobei sein Krümmungsradius dem Krümmungsradius eines gerundeten Übergangs 15 zwischen dem Boden 11 der Pfanne 6 und einer den Boden 11 in seitlicher Richtung begrenzenden Innenwand 16 der Pfanne 6 entspricht.

Der Durchmesser der Ausnehmung 9 entspricht demjenigen des Radiuskopfes 7, so daß die teilsphärische innere Oberfläche der Ausnehmung 9 gegen die Kugeloberfläche des Radiuskopfes 7 anliegt und auf dieser gleitet. Der Radiuskopf 7 ist zusammen mit einem daran angrenzenden schlankeren zylindrischen Hals 17, einem unterhalb des Halses 17 angeordneten scheibenförmigen Kragen 18 mit kreisförmigem Querschnitt und einem an den Kragen 18 angrenzenden Radiusschaft 19 mit einem dem Durchmesser des Halses 17 entsprechenden Durchmesser einstückig aus CoCrMo ausgebildet, so daß die Artikulationsfläche für die Schwenkbewegung, d.h. die teilsphärische innere Oberfläche der Ausnehmung 9 und die Kugeloberfläche des Radiuskopfes 7, die gegeneinander anliegen und aufeinander gleiten, durch die verschleißarme Materialpaarung HDPE/CoCrMo gebildet wird.

Die innere teilsphärische Oberfläche der Ausnehmung 9 ist etwas größer als eine Halbkugel, d.h. ihr Mittelpunktswinkel α in einer Schnittebene durch die Mittelachse 20 des Radiusteils 3 ist größer als 180 Grad, so daß der in die Ausnehmung 9 eingesetzte Radiuskopf 7 in der besagten Schnittebene auf einem größeren Teil seines Umfangs vom Gleitkörper 5 umschlossen und in der Ausnehmung 9 festgehalten wird. Zum Einsetzen des Radiuskopfes 7 in die Ausnehmung 9 wird eine die Ausnehmung 9 umgebende Wand 21 des Gleitkörpers 5 im Bereich einer Austrittsöffnung 22 für den Hals 17 des Radiusteils 3 so weit elastisch aufgeweitet, daß sich der Radiuskopf 7 in der Ausnehmung 9 einrasten läßt, d.h. mit seinem größten Durchmesser durch die Austrittsöffnung 22 hindurchtritt. Um das Einführen des Radiuskopfes 7 in die Ausnehmung 9 zu erleichtern, ist die Austrittsöffnung 22 nach innen zu konisch verjüngt, so daß sich die Wand 21 in diesem Bereich infolge von Keilkräften aufweitet, wenn der Radiuskopf 7 des Radiusteils 3 mit seiner Oberseite von außen in die Austrittsöffnung 22 eingeführt und das Radiusteil 3 dann mit einer axialen Druckkraft beaufschlagt wird. Nach dem Einrasten des Radiuskopfes 7 kehrt die verformte Wand 21 des Gleitkörpers 5 infolge der elastischen Rückstellkraft in ihre Ausgangslage zurück und hält den Radiuskopf 7 in der Ausnehmung 9 fest.

Die bezüglich ihrer Mittelachse 23 rotationssymmetrische zylindrische Innenwand 16 der Pfanne 6 weist einen Durchmesser auf, der etwas größer als der Außendurchmesser der zylindrischen Umfangsfläche 12 des Gleitkörpers 5 ist, so daß bei einer mittigen Anordnung des Radiuskopfes 7 in der Pfanne 6 und bei fluchtenden Mittelachsen 20,23, entsprechend einer Ruhelage des Radiusteils 3 bei unverdrehtem Unterarm, allseitig ein größeres Spiel zwischen der Innenwand 16 der Pfanne 6 und der Umfangsfläche 12 des Gleitkörpers 5 vorhanden ist. Zum Beispiel kann bei einem Innendurchmesser der Pfanne 6 von 13 mm der Außendurchmesser der Umfangsfläche 12 10 mm betragen, so daß das Spiel, d.h. die Breite eines Umfangsspaltes zwischen dem Gleitkörper 5 und der Pfanne 6 in der Ruhelage überall 1,5 mm beträgt. Aufgrund des Spiels kann sich der Gleitkörper 5 parallel zum Boden 11 der Pfanne 6 mit zwei Freiheitsgraden verschieben, wenn der Unterarm um seine Längsachse verdreht wird. Dabei stellt sich der Gleitkörper 5 in der Pfanne 6 den vorgefundenen anatomischen Verhältnissen und der Kipplage des Radiusteils entsprechend ein. Bei entsprechender Implantation des Ellbogengelenkes kann sich der Gleitkörper in seiner Ruhelage bei unverdrehtem Unterarm mittig zur Pfanne 6 einstellen. Die Pfanne 6 kann auch eine von der Rotationssymmetrie abweichende Gestaltung besitzen, beispielsweise kann sie einen elliptischen Rand aufweisen. Entscheidend ist jedoch, daß sich der Gleitkörper 5 an die Innenwand 16 anlehnen kann, ohne sich in der Pfanne 6 zu verklemmen.

Das Verhältnis zwischen der Tiefe der nach unten offenen Pfanne 6 und der Höhe des Gleitkörpers 5 liegt zwischen etwa 1:2 und 2:3, so daß der Gleitkörper 5 zum Teil nach unten über einen ebenen unteren Rand 24 der Pfanne 6 übersteht. Bei einer Höhe des Gleitkörpers 5 von etwa 9 mm kann die Tiefe der Pfanne 6 etwa 5 bis 7 mm betragen und ist damit ausreichend groß, um den Gleitkörper 5 mit Unterstützung des am Hals 17 vorbeigeführten Muskel- und Bänderapparates in der Pfanne 6 zu halten.

Der mit der Pfanne 6 versehene Ausleger 8 ist im wesentlichen senkrecht zu einem Ulnaschaft 25 des Ulnateils 2 und zu der Schwenkebene des Schwenklagers 4 am oberen Ende des Ulnateils 2 angeformt, wobei er in Richtung der Außenseite des Ellbogengelenks seitlich über das Ulnateil 2 übersteht. Der Ausleger 8 weist eine abgeflachte Oberseite 27 mit gerundeten Kanten und auf drei Seiten daran anschließende seitliche Begrenzungsflächen auf, die im wesentlichen senkrecht zur Oberseite 27 sind.

Das Ulnateil 2 und der mit diesem verbundene Ausleger 8 bestehen aus CoCrMo, so daß an der Artikulationsfläche für die Querverschiebung, die von den aufeinander gleitenden Oberflächen der Pfanne 6 bzw. des Gleitkörpers 5, d.h. vom ebenen Pfannenboden 11 und der Oberseite 10 des Gleitkörpers 5 gebildet werden, ebenfalls die verschleißarme Materialpaarung CoCrMo/HDPE vorliegt.

Die anderen Teile der Ellbogengelenk-Endoprothese, d.h. das Schwenklager und das Humerusteil weisen eine Ausbildung auf, wie in der DE OS 39 40 728 beschrieben, deren Offenbarung im Hinblick auf die übrige Ausgestaltung der Prothese, insbesondere auch auf die Abmessungen und Lagebeziehungen von deren Komponenten hiermit als Teil der vorliegenden Anmeldung aufgenommen werden soll.

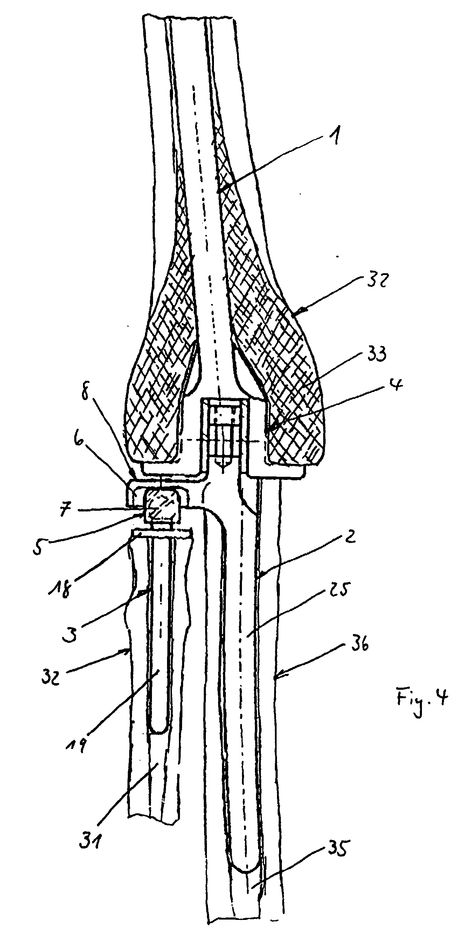

Nach einer Implantation der Ellbogengelenk-Endoprothese liegt der Kragen 18 des Radiusteils 3 mit seiner Unterseite von oben her auf einer Schnittfläche am oberen Ende des abgetrennten Radius 30 auf, während der Radiusschaft 19 im Knochenkanal 31 des Radius 30 verankert ist (Fig. 4). Die Schnittfläche des Radius 30 wird dabei so angeordnet, daß der Abstand zwischen dieser und dem Boden 11 der Pfanne 6 gleich dem Abstand zwischen der Unterseite des Kragens 18 und der Oberseite 10 des auf dem Radiuskopf 7 eingerasteten Gleitkörpers 5 ist.

Das Humerusteil 1 ist im Humerus 32 eingesetzt, dessen aus Spongiosaknochen 33 bestehendes unteres Ende zuvor ausgefräst wurde, um das Schwenklager 4 aufzunehmen, während der Ulnaschaft 25 im Knochenkanal 35 der Ulna 36 verankert ist.

## Patentansprüche

1. Ellbogengelenk-Endoprothese mit einem Humerusteil, einem Ulnateil und einem Radiusteil, bei der das Ulnateil im Bereich des Humerusteils in einem Schwenklager geführt und der Radiuskopf (7) des Radiusteils (3) in einem Gleitkörper (5) verschwenkbar gelagert ist, der im Bereich des Ulnateils in einer Pfanne quer zur Radiusrichtung verschiebbar gehalten ist, **dadurch gekennzeichnet, daß** der Boden (11) der Pfanne (6) und eine dem Boden (11) zugewandte Oberseite (10) des Gleitkörpers (5) im wesentlichen eben sind und die Pfanne (6) eine umlaufende Innenwand (16) als seitlichen Anschlag für den Gleitkörper (5) aufweist.

2. Ellbogengelenk-Endoprothese nach Anspruch 1 **dadurch gekennzeichnet, daß** der Gleitkörper (5) senkrecht zu einer Mittelachse (23) der Pfanne (6) mit zwei Freiheitsgraden beweglich ist.

3. Ellbogengelenk-Endoprothese nach Anspruch 1 oder 2, **dadurch gekennzeichnet, daß** der Gleitkörper (5) zu seiner Mittelachse (20) rotationssymmetrisch ist.

4. Ellbogengelenk-Endoprothese nach einem der Ansprüche 1 bis 3, **dadurch gekennzeichnet, daß** eine Höhe des Gleitkörpers (5) größer oder gleich einer Tiefe der Pfanne (6) ist.

5. Ellbogengelenk-Endoprothese nach einem der Ansprüche 1 bis 4, **dadurch gekennzeichnet, daß** die Innenwand (16) stetig ineinander übergehende Abschnitte aufweist, von denen jeder für eine flächige Anlage des Gleitkörpers (5) vorgesehen ist.

6. Ellbogengelenk-Endoprothese nach Anspruch 5, **dadurch gekennzeichnet, daß** die Innenwand (16) in Form einer Ellipse ausgebildet ist.

7. Ellbogengelenk-Endoprothese nach Anspruch 6, **dadurch gekennzeichnet, daß** die Innenwand (16) rotationssymmetrisch zur Mittelachse (23) der Pfanne (6) ist.

8. Ellbogengelenk-Endoprothese nach einem der Ansprüche 5 bis 7, **gekennzeichnet durch** einen gerundeten Übergang (15) zwischen dem Boden (9) und der Innenwand (16) der Pfanne (6) sowie einen gerundeten Übergang (14) zwischen der Oberseite (10) des Gleitkörpers (5) und dessen seitlichen, der Innenwand (16) der Pfanne (6) zugewandten Umfangsfläche (12).

9. Ellbogengelenk-Endoprothese nach einem der Ansprüche 1 bis 8, **dadurch gekennzeichnet, daß** der Radiuskopf (7) und eine an die Form des Radiuskopfes (7) angepaßte Ausnehmung (9) des Gleitkörpers (5) aneinander angepaßte teilsphärische Oberflächen aufweisen.

10. Ellbogengelenk-Endoprothese nach Anspruch 9, **dadurch gekennzeichnet, daß** die teilsphärische Oberfläche der Ausnehmung (9) etwas größer als die Oberfläche einer Halbkugel ist und der Radiuskopf (7) im Gleitkörper (5) eingeratet ist.

11. Ellbogengelenk-Endoprothese nach einem der Ansprüche 1 bis 10, **dadurch gekennzeichnet, daß** die Pfanne (6) auf der Unterseite eines Auslegers (8) angeordnet ist, der im wesentlichen senkrecht zu einem Ulnaschaft (25) des Ulnateils (2) und einer Schwenkebene des Schwenklagers (4) seitlich über das Ulnateil (2) übersteht.

12. Ellbogengelenk-Endoprothese nach Anspruch 11, **dadurch gekennzeichnet, daß** der Ausleger (8) einstückig am Ulnateil (2) angeformt ist.

13. Ellbogengelenk-Endoprothese nach einem der Ansprüche 1 bis 12, **dadurch gekennzeichnet, daß** das Radiusteil (3) einen an den Radiuskopf (7) angrenzenden zylindrischen Hals (17) aufweist.

14. Ellbogengelenk-Endoprothese nach Anspruch 13, **dadurch gekennzeichnet, daß** das Radiusteil (3) einen zwischen dem Hals (17) und einem Radiusschaft (19) angeordneten Kragen (18) aufweist.

15. Ellbogengelenk-Endoprothese nach Anspruch 14, **dadurch gekennzeichnet, daß** das Radiusteil (3) zu einer Mittelachse des Radiusschaftes (19) rotationssymmetrisch ist.

## Claims

1. An elbow joint endoprosthesis with a humerus part, an ulna part and a radius part, in which the ulna part is guided in the vicinity of the humerus part in a pivot mounting and the radius head (7) of the radius part (3) is pivotably mounted in a slide member (5) which is retained displaceably in the vicinity of the ulna part in a socket transversely to the radius direction, **characterised in that** the bottom (11) of the socket (6) and an upper side (10) of the slide member (5) facing the bottom (11) are substantially plane and the socket (6) has a peripheral inner wall (16) as a lateral abutment for the slide member (5).

2. An elbow joint endoprosthesis according to Claim 1, **characterised in that** the slide member (5) is movable perpendicularly to a medial axis (23) of the socket (6) with two degrees of freedom.

3. An elbow joint endoprosthesis according to Claim 1 or 2, **characterised in that** the slide member (5) is rotationally symmetrical to its medial axis (20).

4. An elbow joint endoprosthesis according to any one of Claims 1 to 3, **characterised in that** one height of the slide member (5) is greater than or equal to one depth of the socket (6).

5. An elbow joint endoprosthesis according to any one of Claims 1 to 4, **characterised in that** the inner wall (16) has portions merging continuously into one another, each of which is provided for planar abutment of the slide member (5).

6. An elbow joint endoprosthesis according to Claim 5, **characterised in that** the inner wall (16) is in the form of an ellipse.

7. An elbow joint endoprosthesis according to Claim 6, **characterised in that** the inner wall (16) is rotationally symmetrical to the medial axis (23) of the socket (6).

8. An elbow joint endoprosthesis according to any one of Claims 5 to 7, **characterised by** a rounded transition (15) between the bottom (9) and the inner wall (16) of the socket (6) as well as a rounded transition (14) between the upper side (10) of the slide member (5) and its lateral peripheral surface (12) facing the inner wall (16) of the socket (6).

9. An elbow joint endoprosthesis according to any one of Claims 1 to 8, **characterised in that** the radius head (7) and a recess (9) of the slide member (5) adapted to the shape of the radius head (7) have mutually adapted partly spherical surfaces.

10. An elbow joint endoprosthesis according to Claim 9, **characterised in that** the partly spherical surface of the recess (9) is slightly larger than the surface of a hemisphere and the radius head (7) is engaged in the slide member (5).

11. An elbow joint endoprosthesis according to any one of Claims 1 to 10, **characterised in that** the socket (6) is arranged on the underside of a side arm (8) which projects substantially perpendicular to an ulna shaft (25) of the ulna part (2) and a pivot plane of the pivot mounting (4) laterally beyond the ulna part (2).

12. An elbow joint endoprosthesis according to Claim 11, **characterised in that** the side arm (8) is formed integrally on the ulna part (2).

13. An elbow joint endoprosthesis according to any one of Claims 1 to 12, **characterised in that** the radius part (3) has a cylindrical neck (17) adjacent the radius head (7).

14. An elbow joint endoprosthesis according to Claim 13, **characterised in that** the radius part (3) has a collar (18) disposed between the neck (17) and a radius shaft (19).

15. An elbow joint endoprosthesis according to Claim 14, **characterised in that** the radius part (3) is rotationally symmetrical to a medial axis of the radius shaft (19).

## Revendications

1. Endoprothèse de l'articulation du coude comprenant une partie humérale, une partie ulnaire et une partie radiale, dans laquelle la partie uinaire est logée dans un palier de pivotement dans la zone de la partie humérale et la tête de radius (7) de la partie radiale (3) est logée de manière pivotante dans un corps coulissant (5), qui est logé de manière mobile transversalement à la direction du radius dans une cavité dans la zone de la partie ulnaire, **caractérisée en ce que** le fond (11) de la cavité (6) et une face supérieure du corps coulissant (5) orientée vers le fond (11) sont sensiblement plans et la cavité (6) comporte une paroi intérieure (16) périphérique formant une butée latérale pour le corps coulissant (5).

2. Endoprothèse de l'articulation du coude selon la revendication 1, **caractérisée en ce que** le corps coulissant (5) est mobile avec deux degrés de liberté perpendiculairement à un axe médian (23) de la cavité (6).

3. Endoprothèse de l'articulation du coude selon la revendication 1 ou 2, **caractérisée en ce que** le corps coulissant (5) présente une symétrie de révolution par rapport à son axe médian (20).

4. Endoprothèse de l'articulation du coude selon l'une quelconque des revendications 1 à 3, **caractérisée en ce qu'**une hauteur du corps coulissant (5) est supérieure ou égale à une profondeur de la cavité (6).

5. Endoprothèse de l'articulation du coude selon l'une quelconque des revendications 1 à 4, **caractérisée en ce que** la paroi intérieure (16) comporte des parties qui s'enchaînent sans discontinuité, chacune d'entre elles étant prévue pour un appui plat du corps coulissant (5).

6. Endoprothèse de l'articulation du coude selon la revendication 5, **caractérisée en ce que** la paroi intérieure (16) est conçue en forme d'ellipse.

7. Endoprothèse de l'articulation du coude selon la revendication 6, **caractérisée en ce que** la paroi intérieure (16) présente une symétrie de révolution par rapport à l'axe médian (23) de la cavité (6).

8. Endoprothèse de l'articulation du coude selon l'une quelconque des revendications 5 à 7, **caractérisée par** une zone de transition (15) arrondie entre le fond (9) et la paroi intérieure (16) de la cavité (6), ainsi qu'une zone de transition (14) arrondie entre la face supérieure (10) du corps coulissant (5) et la face périphérique (12) latérale de celui-ci, orientée vers la paroi intérieure (16) de la cavité (6).

9. Endoprothèse de l'articulation du coude selon l'une quelconque des revendications 1 à 8, **caractérisée en ce que** la tête de radius (7) et un évidement (9), réalisé dans le corps coulissant (5) et adapté à la forme de la tète du radius (7), ont des surfaces partiellement sphériques adaptées les unes aux autres.

10. Endoprothèse de l'articulation du coude selon la revendication 9, **caractérisée en ce que** la surface partiellement sphérique de l'évidement (9) est légèrement supérieure à la surface d'une demi-sphère et la tête du radius (7) est encliquetée dans le corps coulissant (5).

11. Endoprothèse de l'articulation du coude selon l'une quelconque des revendications 1 à 10, **caractérisée en ce que** la cavité (6) est agencée sur la face inférieure d'une saillie (8), qui forme un porte-à-faux latéral sur la partie ulnaire (2) et qui est sensiblement perpendiculaire à une tige de cubitus (25) de la partie ulnaire (2) et à un plan de pivotement du palier de pivotement (4).

12. Endoprothèse de l'articulation du coude selon la revendication 11, **caractérisée en ce que** la saillie (8) est formée d'une seule pièce avec la partie ulnaire (2).

13. Endoprothèse de l'articulation du coude selon l'une quelconque des revendications 1 à 12, **caractérisée en ce que** la partie radiale (3) comporte un col (17) cylindrique adjacent à la tête du radius (7).

14. Endoprothèse de l'articulation du coude selon la revendication 13, **caractérisée en ce que** la partie radiale (3) comporte un collet (18) agencé entre le col (17) et une tige de radius (19).

15. Endoprothèse de l'articulation du coude selon la revendication 14, **caractérisée en ce que** la partie radiale (3) présente une symétrie de révolution par rapport à un axe médian de la tige de radius (19).
